# EUROPÄISCHE PATENTSCHRIFT

(11) **EP 0 910 990 B1**
(45) Veröffentlichungstag und Bekanntmachung des Hinweises auf die Patenterteilung: **23.02.2005**
(21) Anmeldenummer: 98118814.7
(22) Anmeldetag: 05.10.1998
(51) Int. Cl.: A61B 6/08

(54) **Röntgengerät**
X-ray apparatus
Appareil a rayons x

(30) Priorität: 17.10.1997 DE 19746093
(43) Veröffentlichungstag der Anmeldung: 28.04.1999
(73) Patentinhaber: SIEMENS AKTIENGESELLSCHAFT, 80333 München (DE)
(72) Erfinder: Graumann, Rainer Dr., 91315 Höchstadt (DE)

(56) Entgegenhaltungen:
- WO-A-89/10090
- WO-A-91/07913
- DE-U- 9 017 443
- US-A- 4 791 934

## Beschreibung

Die Erfindung betrifft ein Röntgengerät mit einem eine Röntgenstrahlenquelle und eine Röntgenstrahlenempfangseinrichtung aufweisenden Röntgensystem, welches relativ zu einem zu untersuchenden Objekt zur Aufnahme von 2D-Projektionen von einem Bereich des Objektes mit anschließender 3D-Bildrekonstruktion des Bereiches des Objektes verstellbar ist.

Röntgengeräte der eingangs genannten Art weisen zur Aufnahme der Röntgenstrahlenquelle und der Röntgenstrahlenempfangseinrichtung in der Regel einen C-Bogen auf, welcher derart an dem Röntgengerät in einer Halterung gelagert ist, daß er längs seines Umfanges in einem bestimmten Winkelbereich motorisch verstellbar ist (Orbitalbewegung). Zur Gewinnung von 2D-Projektionen aus unterschiedlichen Projektionswinkeln für die 3D-Bildrekonstruktion, beispielsweise eines Körperbereiches eines Lebewesens, mit Hilfe des C-Bogen-Röntgengerätes, wird der C-Bogen nach entsprechender Plazierung relativ zu dem zu untersuchenden Lebewesen bei der Aufnahme der 2D-Projektionen von dem Körperbereich des Lebewesens längs seines Umfanges verstellt. Aus den während der Verstellbewegung des C-Bogens mit dem Röntgensystem aufgenommenen 2D-Projektionen werden anschließend 3D-Bilder des Körperbereiches des Lebewesens rekonstruiert. Die 3D-Bildrekonstruktion setzt allerdings die genaue Kenntnis der Projektionswinkel, d. h. die genaue Position der Röntgenstrahlenquelle und der Röntgenstrahlenempfangseinrichtung während jeder der einzelnen 2D-Projektionen voraus.

Als problematisch erweist es sich, daß bekannte stationäre und ganz besonders mobile C-Bogen-Röntgengeräte mechanische Instabilitäten, insbesondere die Verstellung des C-Bogens längs seines Umfanges betreffend, aufweisen, wodurch Abweichungen der realen Verstellbewegung des C-Bogens von der idealen Verstellbewegung auftreten. Die Bestimmung der Projektionswinkel ist dabei häufig mit Fehlern behaftet, worunter die Qualität der aus den 2D-Projektionen rekonstruierten 3D-Bilder leidet.

Zur Vermeidung von Fehlern bei der Bestimmung der Projektionswinkel sind folgende zwei Methoden bekannt:
a) Aus der DE 195 12 819 A1 ist die Verwendung eines in der Regel aus Plexiglas mit eingesetzten Metallstrukturen gebildeten Markerrings bekannt, welcher um den zu untersuchenden Körperbereich des Lebewesens angeordnet wird. In den 2D-Projektionen des zu untersuchenden Körperbereiches sind die Metallstrukturen des Markerrings sichtbar, so daß aus deren Position die jeweiligen Projektionswinkel der 2D-Projektionen berechenbar sind. Dieses Verfahren hat jedoch den Nachteil, daß der Markerring einen relativ großen Durchmesser aufweist, so daß der Abstand zwischen Röntgenstrahlenquelle und Markerring sehr klein ist (wenige Zentimeter) . Die Metallstrukturen werden daher mit sehr großer Vergrößerung in den 2D-Projektionen abgebildet, so daß große Teile der 2D-Projektionen von den Metallstrukturen überlagert sind. Des weiteren wird nur ein kleiner Bereich der Metallstrukturen des Markerrings in den 2D-Projektionen abgebildet, so daß die Bestimmung der Projektionswinkel anhand der geringen Anzahl von abgebildeten Metallstrukturen schwierig ist.
b) Eichmessungen vor der eigentlichen Patientenmessung unter der Annahme, daß das Systemverhalten, d. h. im wesentlichen die Verstellbewegungen des C-Bogens, in hohem Maße reproduzierbar ist. Dieses Verfahren ist jedoch sehr zeitaufwendig und zudem nur bei mechanisch verstärkten stationären C-Bogen-Röntgengeräten anwendbar. Die Anwendung auf mobile Röntgengeräte ist wegen der bereits erwähnten mechanischen Instabilität derartiger Röntgengeräte nicht möglich, wobei mechanische Stabilisierungen aufgrund der großen, die Mobilität einschränkenden Gewichtszunahme für mobile Röntgengeräte ausgeschlossen sind.

Aus der US 5,109,397 ist ein mobil ausgeführter Computertomograph bekannt, dessem um ein Rotationszentrum rotierenden eine Röntgenstrahlenquelle und eine Röntgenstrahlenempfangseinrichtung aufweisenden Röntgensystem Sensoren zugeordnet sind, welche sich mit dem Röntgensystem mitbewegen und zur Detektion seitlicher Bewegungen des Röntgensystems während eines Scans mit einem um das Rotationszentrum angeordneten, ortsfesten Ring zusammenwirken. Die Sensoren erzeugen dabei Signale, deren Auswertung die Ermittlung der Abstände zwischen ihrem definierten Anbringungsort und dem Ring gestatten. Die gewonnenen Daten werden anschließend bei der Rekonstruktion von Schnittbildern herangezogen. Der Ring ist dabei im Ausbreitungsweg eines von der Röntgenstrahlenquelle ausgehenden Röntgenstrahlenbündels angeordnet.

Sensoren zur Ermittlung des Abstandes zweier Objekte voneinander sind auch aus der DE 43 32 254 C1 und der DE 94 08 562 U1 bekannt. Die Ermittlung des Abstandes erfolgt dabei durch die Laufzeitmessung von reflektierten Schallwellen oder elektromagnetischen Wellen.

Aus der DE 36 04 955 A1 ist ein Röntgendiagnostikgerät mit einem Bilderzeugungssystem mit Röntgenstrahler und Strahlenempfänger sowie einem Lagerungstisch bekannt. Mit den verstellbaren Komponenten des Bilderzeugungssystems sind Positionsgeber in Form von Potentiometern verbunden, welche die Stellung dieser Komponenten erfassen.

In der DE 195 35 583 A1 ist außerdem ein Röntgendiagnostikgerät mit einer Positionierhilfe beschrieben. An einem Röntgenbildverstärker ist dabei ein Lichtsender zum Aussenden eines Lichtbündels derart vorgesehen, daß dieses auf einen den Röntgenbildverstärker gegenüberliegenden Röntgenstrahler fokussiert ist. Auf diese Weise kann eine Positionierung von Röntgenstrahler und Röntgenbildverstärker in bezug auf ein Untersuchungsobjekt ohne die Verwendung von Röntgenstrahlung erfolgen.

Aus der DE 90 17 433 U1 ist eine Vorrichtung zur Bestimmung von Lage und Orientierung eines Röntgen-C-Bogens gegenüber einem Lithotripter bekannt. An einem der beiden Geräte sind mindestens drei Schallquellen und am anderen Gerät mindestens drei Schallaufnehmer angebracht. Mittels eines Computers wird aufgrund der Schallaufzeiten die Lage und Orientierung der beiden Geräte zueinander ermittelt.

Der Erfindung liegt die Aufgabe zugrunde, ein Röntgengerät der eingangs genannten Art derart auszuführen, daß die Ermittlung der Projektionswinkel vereinfacht und sowohl für stationäre als auch mobile Röntgengeräte geeignet ist.

Nach der Erfindung wird diese Aufgabe gelöst durch ein Röntgengerät mit einem eine Röntgenstrahlenquelle und eine Röntgenstrahlenempfangseinrichtung aufweisenden Röntgensystem, welches relativ zu einem zu untersuchenden Objekt zur Aufnahme von 2D-Projektionen von einem Bereich des Objektes mit anschließender 3D-Bildrekonstruktion des Bereiches des Objektes verstellbar ist, wobei das Röntgengerät zur Ermittlung von für die 3D-Bildrekonstruktion erforderlichen, zu den einzelnen 2D-Projektionen gehörigen Projektionswinkeln Sendeund Empfangseinrichtungen für Schallwellen aufweist, wobei die Sende- oder die Empfangseinrichtungen mit dem Röntgensystem mitbewegt werden und im Bereich der Röntgenstrahlenquelle und der Röntgenstrahlenempfangseinrichtung angeordnet sind und die Empfangs- oder die Sendeeinrichtungen an in bezug auf das Röntgensystem stationären Teilen des Röntgengerätes angeordnet sind und wobei das Röntgengerät Mittel zur Ermittlung der Projektionswinkel und zur 3D-Bildrekonstruktion aufweist. Beispielsweise sind an stationären Teilen des Röntgengerätes Empfangseinrichtungen und im Bereich der Röntgenstrahlenquelle und der Röntgenstrahlenempfangseinrichtung Sendeeinrichtungen, welche im Zuge einer Verstellbewegung der Röntgenstrahlenquelle und der Röntgenstrahlenempfangseinrichtung mit diesen mitbewegt werden, angeordnet. Während der Verstellbewegung des Röntgensystems mit gleichzeitiger Aufnahme von 2D-Projektionen aus unterschiedlichen Projektionswinkeln senden die Sendeeinrichtungen pro Aufnahme einer 2D-Projektion Schallwellen aus, die von den Empfangseinrichtungen empfangen werden. Die Auswertung der empfangenen Schallwellen ermöglicht anschließend die Bestimmung der genauen Position der Röntgenstrahlenquelle und der Röntgenstrahlenempfangseinrichtung während der Aufnahme einer entsprechenden 2D-Projektion, so daß aus den jeweils ermittelten Positionen der Röntgenstrahlenquelle und der Röntgenstrahlenempfangseinrichtung die Projektionswinkel jeder der 2D-Projektionen berechenbar sind. Die Sende- und Empfangseinrichtungen zur Bestimmung der Projektionswinkel der 2D-Projektionen sind aufgrund ihres geringen Eigengewichts und ihrer geringen Baugröße sowohl in stationären als auch mobil ausgeführten Röntgengeräten einsetzbar, so daß auch mobilen Röntgengeräten der Anwendungsbereich der 3D-Bildgebung offensteht.

Eine Variante der Erfindung sieht vor, daß die Sendeund/oder Empfangseinrichtungen eine derartige Richtcharakteristik aufweisen, daß die von den Sendeeinrichtungen ausgesandten Schallwellen während der Verstellbewegung des Röntgensystems von jeweils mindestens zwei Empfangseinrichtungen empfangen werden. Durch die Verwendung von Sendeeinrichtungen mit Richtcharakteristik wird die erforderliche Sendeleistung der Sendeeinrichtungen im Vergleich zu Sendeeinrichtungen ohne Richtcharakteristik (isotrope Strahler) reduziert. Durch die Verwendung von Empfangseinrichtungen mit Richtcharakteristik wird der Empfang von Störsignalen vermindert, was die Signalverarbeitung vereinfacht.

Gemäß einer besonders bevorzugten Ausführungsform der Erfindung weist das Röntgengerät eine Steuer- und Recheneinheit auf, welche die Phasen und/oder Laufzeiten der Schallwellen zwischen Sende- und Empfangseinrichtungen zur Bestimmung der Projektionswinkel ermittelt. Die Phasen- und/oder Laufzeitinformationen werden dabei derart ausgewertet, daß die Weglängen zwischen Sendeeinrichtungen und Empfangseinrichtungen mit einer Genauigkeit von λ/4 bis λ/8 der Sendewellenlänge nach an sich bekannten Berechnungsverfahren für jede Position der Röntgenstrahlenquelle und der Röntgenstrahlenempfangseinrichtung bei einer 2D-Projektion bestimmbar sind. Zu den aus den Weglängen bestimmbaren Positionen der Röntgenstrahlenquelle und der Röntgenstrahlenempfangseinrichtung pro 2D-Projektion werden anschließend die Projektionswinkel der jeweiligen 2D-Pro-jektionen für die Rekonstruktion von 3D-Bildern gewonnen. Die Positionen der Röntgenstrahlenquelle und der Röntgenstrahlenempfangseinrichtung werden beispielsweise bezüglich eines ortsfesten kartesischen Koordinatensystems berechnet.

Gemäß einer Variante der Erfindung senden die Sendeeinrichtungen Schallwellen unterschiedlicher Frequenz aus. Auf diese Weise ist eine eindeutige Identifizierung der Herkunft einer empfangenen Schallwelle, d. h. von welcher Sendeeinrichtung die Welle ausgesandt wurde, möglich, so daß auch die zur Bestimmung des Projektionswinkels erforderliche Bestimmung der Weglänge zwischen einer Sende- und Empfangseinrichtung verwechslungssicher und exakt erfolgen kann.

Eine weitere Variante der Erfindung sieht eine Einheit zur Messung der Umgebungstemperatur vor, da beispielsweise die Laufzeit von Schallwellen von der Umgebungstemperatur abhängig ist. Auf diese Weise ist die jeweils herrschende Umgebungstemperatur bekannt, so daß in Abhängigkeit von der jeweiligen Umgebungstemperatur die einer Laufzeit des Schalls entsprechende Weglänge exakt bestimmbar ist.

Gemäß einer bevorzugten Ausführungsform der Erfindung sind die Sendeeinrichtungen Ultraschallsender und die Empfangseinrichtungen Ultraschallempfänger, die preisgünstig am Markt erhältlich sind. Ihre Signale sind für die Positionsbestimmung der Röntgenstrahlenquelle und der Röntgenstrahlenempfangseinrichtung derart auswertbar, daß die Projektionswinkel für die Rekonstruktion von 3D-Bildern mit ausreichender Genauigkeit bestimmbar sind.

Gemäß einer Ausführungsform der Erfindung sind die Röntgenstrahlenquelle und die Röntgenstrahlenempfangseinrichtung an den Enden eines C-Bogens angeordnet, wobei die Röntgenstrahlenquelle und die Röntgenstrahlenempfangseinrichtung je eine Sendeeinrichtung und das Röntgengerät mehrere verteilt angeordnete Empfangseinrichtungen aufweist. Die Anordnung je einer Sendeeinrichtung an der Röntgenstrahlenquelle und der Röntgenstrahlenempfangseinrichtung ist dabei für die Bestimmung der die Position der Röntgenstrahlenquelle und der Röntgenstrahlenempfangseinrichtung bei einer 2D-Projektion angebenden räumlichen Koordinaten in dem ortsfesten Koordinatensystem ausreichend.

Ein Ausführungsbeispiel der Erfindung ist in der beigefügten schematischen Zeichnung dargestellt, welche ein erfindungsgemäßes Röntgengerät zeigt.

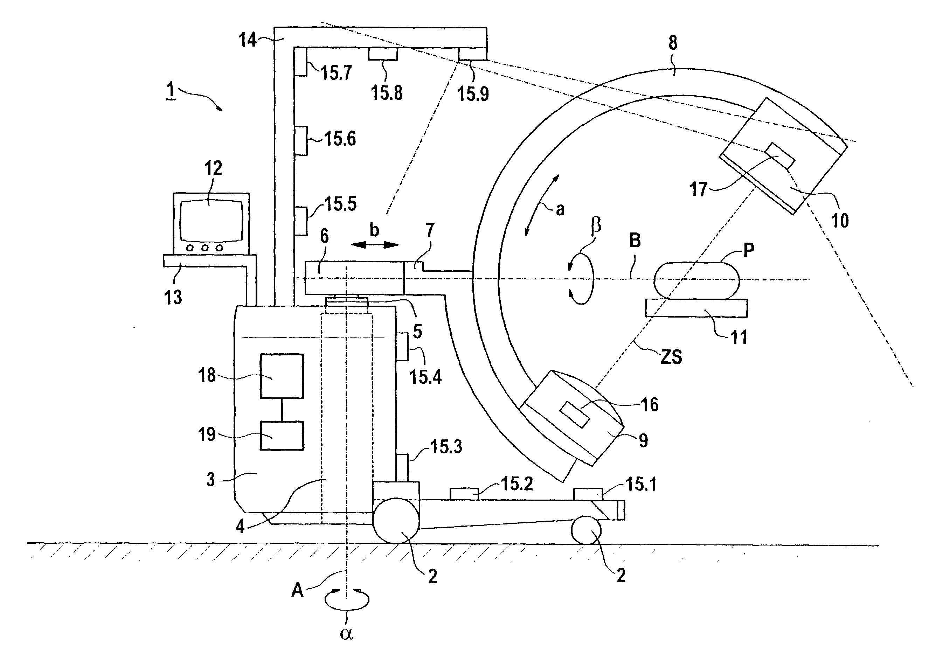

Im Falle des vorliegenden Ausführungsbeispiels handelt es sich bei dem erfindungsgemäßen Röntgengerät um ein C-Bogen-Röntgengerät 1 mit einem auf Rädern 2 verfahrbaren Gerätewagen 3. Das C-Bogen-Röntgengerät 1 weist eine in der Figur nur schematisch angedeutete Hubvorrichtung 4 mit einer eine Längsachse A aufweisenden Säule 5, um die die Säule 5 in Richtung des Doppelpfeiles α drehbar ist, auf. An der Säule 5 ist ein Halteteil 6 angeordnet, an dem wiederum ein Lagerteil 7 zur Lagerung eines C-Bogens 8 angeordnet ist. Der C-Bogen 8 weist an seinen beiden Enden einander gegenüberliegend eine Röntgenstrahlenquelle 9 und eine Röntgenstrahlenempfangseinrichtung 10 auf, welche derart relativ zueinander angeordnet sind, daß ein von der Röntgenstrahlenquelle 9 ausgehender Zentralstrahl ZS eines Röntgenstrahlenbündels annähernd mittig auf die Röntgenstrahlenempfangseinrichtung 10 trifft. Der C-Bogen 8 ist in an sich bekannter Weise in Richtung des Doppelpfeiles a längs seines Umfanges in nicht näher dargestellter Weise motorisch verstellbar an dem Lagerteil 7 gelagert. Das Lagerteil 7 ist in an sich bekannter Weise um eine gemeinsame Achse B des Halteteils 6 und des Lagerteils 7 drehbar (vgl. Doppelpfeil β, Angulation) und in Richtung der Achse B verschieblich (vgl. Doppelpfeil b) an dem Halteteil 6 gelagert. Mit Hilfe der Hubvorrichtung 4 ist der C-Bogen 8, der über das Lagerteil 7 und das Halteteil 6 mit der Säule 5 der Hubvorrichtung 4 verbunden ist, relativ zu dem Gerätewagen 3 vertikal verstellbar.

Das C-Bogen-Röntgengerät 1 ist im Falle des vorliegenden Ausführungsbeispiels zur Erzeugung von 3D-Bildern eines Körperbereiches eines in der Figur nur schematisch dargestellten, auf einer Patientenliege 11 liegenden Patienten P vorgesehen. Die 3D-Bilder werden aus 2D-Projektionen des Körperbereiches aus unterschiedlichen Projektionswinkeln, welche mit Hilfe des die Röntgenstrahlenquelle 9 und die Röntgenstrahlenempfangseinrichtung 10 aufweisenden Röntgensystems gewonnen werden, rekonstruiert und sind mittels eines Monitors 12, welcher auf einem Halter 13 des C-Bogen-Röntgengerätes 1 angeordnet ist, darstellbar.

Zur Aufnahme von 2D-Projektionen aus unterschiedlichen Projektionswinkeln wird der das Röntgensystem aufnehmende C-Bogen 8 längs seines Umfanges in Richtung des Doppelpfeiles a in einem Winkelbereich von ca. 200° um den zu untersuchenden und darzustellenden Körperbereich des Patienten P motorisch verstellt, wobei während der Verstellbewegung ca. 50 bis 100 2D-Projektionen von dem Körperbereich des Patienten P mit dem Röntgensystems aus unterschiedlichen Projektionswinkeln aufgenommen werden.

Zur exakten Ermittlung der unterschiedlichen Projektionswinkel der 2D-Projektionen, welche für die 3D-Bildrekonstruktion des Körperbereiches des Patienten aus den 2D-Projektionen unbedingt erforderlich sind, weist das C-Bogen-Röntgengerät 1 mehrere an dem in bezug auf den C-Bogen 8 stationären Gerätewagen 3 und einer stationären Haltevorrichtung 14 des Gerätewagens 3 angeordnete Empfangseinrichtungen auf. Im Falle des vorliegenden Ausführungsbeispiels handelt es sich bei den Empfangseinrichtungen um Ultraschallempfänger 15.1 bis 15.9. Die Röntgenstrahlenquelle 9 sowie die Röntgenstrahlenempfangseinrichtung 10 sind jeweils mit einer Sendeeinrichtung in Form eines Ultraschallsenders 16 und 17 versehen, welche bei der Verstellbewegung des C-Bogens 8 längs seines Umfanges mit dem Röntgensystem mitbewegt werden. Die Ultraschallempfänger 15.1 bis 15.9 sind dabei derart an dem Gerätewagen 3 und der Haltevorrichtung 14 angebracht, daß während der Verstellbewegung des C-Bogens 8 eine frei Sichtlinie der Ultraschallsender 16, 17 zu jeweils mindestens zwei der Ultraschallempfänger 15.1 bis 15.9 vorhanden ist.

Eine Steuer- und Recheneinheit 18 des C-Bogen-Röntgengerätes 1, welche sowohl die motorische Verstellbewegung des C-Bogens 8 steuert als auch die Aufnahme von 2D-Projektionen auslöst, steuert auch die Auslösung von Ultraschallwellen der Ultraschallsender 16 und 17, welche jeweils gleichzeitig mit der Auslösung einer 2D-Projektion erfolgt. Auf diese Weise ist für jede 2D-Projektion die entsprechende Position der Röntgenstrahlenquelle 9 und der Röntgenstrahlenempfangseinrichtung 10 und somit der jeweilige Projektionswinkel ermittelbar. Die Signalleitungen zur Übertragung der Steuersignale für die motorische Verstellung des C-Bogens 8, die Auslösung von 2D-Projektionen und die Auslösung von Ultraschallwellen sind in der Figur im übrigen nicht dargestellt.

Die Ultraschallsender 16 und 17 werden mit unterschiedlichen Frequenzen im Bereich von ca. 200 kHz betrieben, so daß die von den Ultraschallempfängern 15.1 bis 15.9 empfangenen Ultraschallwellen eindeutig einem der Ultraschallsender 16 oder 17 zugeordnet werden können.

Die Ultraschallsender 16, 17 und die Ultraschallempfänger 15.1 bis 15.9 weisen an die Verstellbewegung des Röntgensystems angepaßte Richtcharakteristiken auf, welche für den Ultraschallempfänger 15.9 und den Ultraschallsender 17 exemplarisch in die Figur mit strichpunktierten Linien eingetragen sind. Auf diese Weise kann der Empfang von die Signalauswertung störenden Fremdsignalen seitens der Ultraschallempfänger 15.1 bis 15.9 vermindert und die erforderliche Sendeleistung der Ultraschallsender 16, 17 gegenüber isotrop strahlenden Ultraschallsendern reduziert werden.

Zur Ermittlung der jeweiligen Positionen der Röntgenstrahlenquelle 9 und der Röntgenstrahlenempfangseinrichtung 10 bei 2D-Projektionen in bezug auf ein ortsfestes beispielsweise kartesisches Koordinatensystem ist es erforderlich, daß jeweils mindestens zwei der Ultraschallempfänger 15.1 bis 15.9 Ultraschallwellen der der Röntgenstrahlenquelle 9 und der Röntgenstrahlenempfangseinrichtung 10 zugeordneten Ultraschallsender 16 und 17 während der Verstellbewegung des C-Bogens 8 empfangen.

Die von den Ultraschallempfängern 15.1 bis 15.9 empfangenen Ultraschallwellen werden anschließend der Steuer- und Recheneinheit 18 zugeführt, welche die Phasen und/oder die Laufzeiten der jeweiligen zu einer 2D-Projektion gehörigen Ultraschallwellen ermittelt. Da die Laufzeit des Ultraschalls abhängig von der Umgebungstemperatur ist, weist das C-Bogen-Röntgengerät 1 außerdem eine an die Steuer- und Recheneinheit 18 angeschlossene Einheit 19 zur Messung der Umgebungstemperatur auf, welche bei der Ermittlung der Laufzeit des Ultraschalls in Ausgleichsrechnungen berücksichtigt wird.

Anhand der Phasen- und/oder Laufzeitinformationen der empfangenen Ultraschallwellen errechnet die Steuer- und Recheneinheit 18 für jede 2D-Projektion die Weglängen zwischen der Röntgenstrahlenquelle 9 bzw. der Röntgenstrahlenempfangseinrichtung 10 und mindestens zwei der als Bezugspunkte fungierenden Ultraschallempfänger 15.1 bis 15.9, welche die entsprechenden Ultraschallwellen empfangen haben. Anhand der während der Verstellbewegung des C-Bogens 8 ermittelten Weglängen und der in dem ortsfesten Koordinatensystem bekannten Positionen der Ultraschallempfänger 15.1 bis 15.9 ist für jede 2D-Projektion die jeweils zugehörige Position der Röntgenstrahlenquelle 9 und der Röntgenstrahlenempfangseinrichtung 10 in bezug auf das ortsfeste Koordinatensystem und somit der zu jeder 2D-Projektion gehörige und für die Rekonstruktion von 3D-Bildern erforderliche Projektionswinkel exakt ermittelbar. Die derart ermittelten Projektionswinkel werden dann zur Rekonstruktion von 3D-Bildern von Körperbereichen des Patienten P herangezogen, welche, wie bereits erwähnt, auf dem Monitor 12 des C-Bogen-Röntgengerätes 1 darstellbar sind.

Die Position der Röntgenstrahlenquelle 9 und der Röntgenstrahlenempfangseinrichtung 10 kann dabei nach an sich bekannten Berechnungsverfahren mit einer Genauigkeit von λ/4 bis λ/8 der verwendeten Ultraschallwellenlänge bestimmt werden. Bei Verwendung von Ultraschall mit einer Frequenz von 200 kHz und einer Wellenlänge der Ultraschallwellen von etwa λ = 1,7 mm, beträgt die Genauigkeit der Positionsbestimmung also 0,425 bis 0,2125 mm.

Die in der Figur eingetragene Richtcharakteristik des Ultraschallempfängers 15.9 und des Ultraschallsenders 17 ist nur exemplarisch zu verstehen. Jeder der Ultraschallsender 16, 17 oder der Ultraschallempfänger 15.1 bis 15.9 kann auch eine andere Richtcharakteristik, welche vorzugsweise der Verstellbewegung des Röntgensystems bei 2D-Projektionen angepaßt ist, aufweisen.

Die Anzahl der zur Positionsbestimmung der Röntgenstrahlenquelle 9 und der Röntgenstrahlenempfangseinrichtung 10 vorgesehenen Ultraschallsender und Ultraschallempfänger kann von der im vorliegenden Ausführungsbeispiel verwendeten Anzahl abweichen. Des weiteren ist die Anbringung der Ultraschallsender und der Ultraschallempfänger an dem C-Bogen-Röntgengerät 1 nur exemplarisch zu verstehen und kann auch anders ausgeführt sein. Insbesondere können die Empfangseinrichtungen auch an der Röntgenstrahlenquelle 9 und der Röntgenstrahlenempfangseinrichtung 10 und die Sendeeinrichtungen an dem Gerätewagen 3 und der Haltevorrichtung 14 angeordnet sein.

Der C-Bogen 8 mit den Ultraschallsendern 16 und 17 kann zur Untersuchung von Körperbereichen eines Lebewesens aus einer anderen als der im vorliegenden Ausführungsbeispiel beschriebenen Stellung auch um die Achse B gekippt (Angulation) und mittels der Hubvorrichtung 4 um die Achse A gedreht werden. Um in diesem Fall ebenfalls die Projektionswinkel während der 2D-Projektionen, d. h. der Verstellbewegung des C-Bogens 8 längs seines Umfanges, bestimmen zu können, d. h. den Empfang der Ultraschallwellen sicherzustellen, müssen aufgrund der Richtcharakteristik der Ultraschallsender 16, 17 und der Ultraschallempfänger 15.1 bis 15.9 auch die Ultraschallempfänger 15.1 bis 15.9 entsprechend der Kippung und Drehung des C-Bogens 8 gekippt und gedreht werden. In diesem Fall ist beispielsweise die Halterung 14 derart auszuführen, daß sie dem Kippen und Drehen des C-Bogens 8 folgen kann.

Im Falle des vorliegenden Ausführungsbeispiels ist die Röntgenstrahlenquelle 9 und die Röntgenstrahlenempfangseinrichtung 10 mit jeweils nur einem Ultraschallsender 16, 17 versehen, wodurch die Bestimmung der Positionen der Röntgenstrahlenquelle 9 und der Röntgenstrahlenempfangseinrichtung 10 in der Orbitalebene (Ebene der Verstellbewegung des C-Bogen) ermöglicht wird. Werden die Röntgenstrahlenquelle 9 und die Röntgenstrahlenempfangseinrichtung 10 mit mindestens zwei Ultraschallsendern versehen, so ist auch die Bestimmung der Neigungswinkel der Röntgenstrahlenquelle 9 und der Röntgenstrahlenempfangseinrichtung 10 in Angulationsrichtung in bezug auf das in der Figur nicht dargestellte Isozentrum des C-Bogens 8 möglich. Derartige Neigungswinkel treten aufgrund der bereits erwähnten Instabilitäten des C-Bogen-Röntgengerätes, insbesondere während der Verstellbewegung des C-Bogens 8 auf.

Anstelle der Ultraschallsender 16, 17 und der Ultraschallempfänger 15.1 bis 15.9 können zur Positionsbestimmung der Röntgenstrahlenquelle 9 und der Röntgenstrahlenempfangseinrichtung 10 Sender und Empfänger vorgesehen sein, die auf Basis anderer Schallwellen arbeiten.

Die Erfindung wurde vorliegend am Beispiel eines mobilen C-Bogen-Röntgengerätes 1 erläutert. Die Verwendung der Sendeund Empfangseinrichtungen zur Positionsbestimmung der Röntgenstrahlenquelle 9 und der Röntgenstrahlenempfangseinrichtung 10 ist jedoch nicht auf den Einsatz in mobilen C-Bogen-Röntgengeräten beschränkt, sondern auch in stationären Röntgengeräten möglich.

## Patentansprüche

1. Röntgengerät mit einem eine Röntgenstrahlenquelle (9) und eine Röntgenstrahlenempfangseinrichtung (10) aufweisenden Röntgensystem, welches relativ zu einem zu untersuchenden Objekt (P) zur Aufnahme von 2D-Projektionen von einem Bereich des Objektes (P) mit anschließender 3D-Bildrekonstruktion des Bereiches des Objektes (P) verstellbar ist, wobei das Röntgengerät zur Ermittlung von für die 3D-Bildrekonstruktion erforderlichen, zu den einzelnen 2D-Projektionen gehörigen Projektionswinkeln Sende- und Empfangseinrichtungen (15.1 bis 15.9, 16, 17) für Schallwellen aufweist, wobei die Sende- oder die Empfangseinrichtungen (15.1 bis 15.9, 16, 17) mit dem Röntgensystem mitbewegt werden und im Bereich der Röntgenstrahlenquelle (9) und der Röntgenstrahlenempfangseinrichtung (10) angeordnet sind und die Empfangsoder die Sendeeinrichtungen (15.1 bis 15.9, 16, 17) an in bezug auf das Röntgensystem stationären Teilen (3) des Röntgengerätes angeordnet sind und wobei das Röntgengerät Mittel (18) zur Ermittlung der Projektionswinkel und zur 3D-Bildrekonstruktion aufweist.

2. Röntgengerät nach Anspruch 1, bei dem die Sende- und Empfangseinrichtungen (15.1 bis 15.9, 16, 17) eine derartige Richtcharakteristik aufweisen, daß die von den Sendeeinrichtungen (16, 17) ausgesandten Schallwellen selbst während der Verstellbewegung des Röntgensystems von jeweils mindestens zwei Empfangseinrichtungen (15.1 bis 15.9) empfangen werden.

3. Röntgengerät nach einem der Ansprüche 1 oder 2, welches eine Steuer- und Recheneinheit (18) aufweist, welche die Phasen und/oder Laufzeiten der Schallwellen zwischen Sende- und Empfangseinrichtungen (15.1 bis 15.9, 16, 17) zur Bestimmung der Projektionswinkel ermittelt.

4. Röntgengerät nach einem der Ansprüche 1 bis 3, bei dem die Sendeeinrichtungen (16, 17) Schallwellen unterschiedlicher Frequenz aussenden.

5. Röntgengerät nach einem der Ansprüche 1 bis 4, bei dem eine Einheit (19) zur Messung der Umgebungstemperatur vorgesehen ist.

6. Röntgengerät nach einem der Ansprüche 1 bis 5, bei dem die Sendeeinrichtungen Ultraschallsender (16, 17) und die Empfangseinrichtungen Ultraschallempfänger (15.1 bis 15.9) sind.

7. Röntgengerät nach einem der Ansprüche 1 bis 6, bei dem die Röntgenstrahlenquelle (9) und die Röntgenstrahlenempfangseinrichtung (10) an den Enden eines C-Bogens (8) angeordnet sind, wobei die Röntgenstrahlenquelle (9) und die Röntgenstrahlenempfangseinrichtung (10) je eine Sendeeinrichtung (16, 17) und das Röntgengerät mehrere verteilt angeordnete Empfangseinrichtungen (15.1 bis 15.9) aufweist.

## Claims

1. X-ray device with an X-ray apparatus having an X-ray source (9) and an X-ray receiver (10) that is adjustable relative to an examination subject (P) for registering 2D projections of a region of the subject (P), with subsequent 3D image reconstruction of the region of the subject (P), wherein the X-ray system for determining projection angles belonging to the individual 2D projections that are required for the 3D image reconstruction has transmission and reception devices (15.1 to 15.9, 16, 17) for acoustic waves, and wherein the transmission or the reception devices (15.1 to 15.9, 16, 17) are co-moved with the X-ray system and are arranged in the region of the X-ray source (9) and the X-ray receiver (10), and the reception devices or the transmission devices (15.1 to 15.9, 16, 17) can be arranged at stationary parts (3) of the X-ray apparatus with reference to the X-ray system and whereby the X-ray device has means (18) for determining the projection angle and the 3D image reconstruction.

2. An X-ray device according to Claim 1, wherein the transmission and reception devices (15.1 to 15.9, 16, 17) have a directional characteristic such that the acoustic waves emitted by the transmission devices (16, 17) are only received by at least two reception devices (15.1 to 16.9 )during the adjustment motion movement of the X-ray system.

3. X-ray device according to one of Claims 1 or 2, having a control and calculating unit (18) which determines the phase and/or transit time of the acoustic waves between the transmission and reception devices (15.1 to 15.9, 16,17) in order to determine the projection angle.

4. X-ray device according to one of Claims 1 to 3, wherein the transmission device (16, 17) emits acoustic waves with different frequencies.

5. X-ray device according to one of Claims 1 to 4, wherein a unit (19) is provided for measuring the ambient temperature.

6. X-ray device according to one of Claims 1 to 5, wherein the transmission device is an ultrasound transmitter (16, 17) and the reception device an ultrasound receiver (15.1 to 15.9).

7. X-ray device according to one of Claims 1 to 6, wherein the X-ray source (9) and the X-ray receiver (10) are arranged on the ends of a C-Arm (8), the X-ray source (9) and the X-ray receiver (10) each having a transmission device (16, 17) and the X-ray device having a plurality of reception devices (15.1 to 15.9) arranged in a distributed manner.

## Revendications

1. Appareil à rayons X comprenant un système à rayons X ayant une source (9) de rayons X et un dispositif (10) de réception de rayons X qui peut être déplacé par rapport à un objet (P) à étudier pour l'enregistrement de projections en deux dimensions d'une région de l'objet (P) avec ensuite reconstruction d'une image en trois dimensions de la région de l'objet (P), l'appareil à rayons X ayant, pour la détermination d'angles de projection nécessaires pour la reconstruction de l'image en trois dimensions et appartenant aux projections en deux dimensions, des dispositifs (15.1 à 15.9, 16, 17) d'émission et de réception d'ondes sonores, les dispositifs (15.1 à 15.9, 16, 17) d'émission ou de réception étant déplacés avec le système à rayons X et étant disposés dans la région de la source (9) de rayons X et du dispositif (10) de réception de rayons X et les dispositifs (15.1 à 15.9, 16, 17) de réception ou d'émission étant disposés sur des parties (3) de l'appareil à rayons X qui sont fixes par rapport au système à rayons X et l'appareil à rayons X ayant des moyens (18) de détermination des angles de projection et pour la reconstruction de l'image en trois dimensions.

2. Appareil à rayons X suivant la revendication 1, dans lequel les dispositifs (15.1 à 15.9, 16, 17) d'émission et de réception ont une caractéristique directionnelle telle que les ondes sonores émises par les dispositifs (16, 17) d'émission sont reçues même pendant le mouvement de déplacement du système à rayons X par, respectivement, au moins deux dispositifs (15.1 à 15.9) de réception.

3. Appareil à rayons X suivant l'une des revendications 1 ou 2, qui a une unité (18) de commande et de calcul qui détermine pour la détermination des angles de projection les phases et/ou les temps de propagation des ondes sonores entre les dispositifs (15.1 à 15.9, 16, 17) d'émission et de réception.

4. Appareil à rayons X suivant l'une des revendications 1 à 3, dans lequel les dispositifs (16, 17) d'émission émettent des ondes sonores de fréquences différentes.

5. Appareil à rayons X suivant l'une des revendications 1 à 4, dans lequel il est prévu une unité (19) de mesure de la température ambiante.

6. Appareil à rayons X suivant l'une des revendications 1 à 5, dans lequel les dispositifs d'émission sont des émetteurs (16, 17) d'ultrasons et les dispositifs de réception sont des récepteurs (15.1 à 15.9) d'ultrasons.

7. Appareil à rayons X suivant l'une des revendications 1 à 6, dans lequel la source (9) de rayons X et le dispositif (10) de réception de rayons X sont disposés aux extrémités d'un arceau (8) en C, la source (9) de rayons X et le dispositif (10) de réception de rayons X ayant, respectivement, un dispositif (16, 17) d'émission et l'appareil à rayons X ayant plusieurs dispositifs (15.1 à 15.9) de réception qui sont répartis.
